# EUROPÄISCHE PATENTSCHRIFT

(11) **EP 1 511 562 B1**
(45) Veröffentlichungstag und Bekanntmachung des Hinweises auf die Patenterteilung: **10.10.2018**
(21) Anmeldenummer: 03725106.3
(22) Anmeldetag: 26.04.2003
(51) Int. Cl.: B01J 23/46, B01J 21/06, B01J 35/10, C07C 5/10

(54) **VERFAHREN ZUR HYDRIERUNG VON AROMATISCHEN VERBINDUNGEN**
METHOD FOR HYDROGENATING AROMATIC COMPOUNDS
PROCEDE D'HYDROGENATION DE COMPOSES AROMATIQUES

(30) Priorität: 10.06.2002 DE 10225565
(43) Veröffentlichungstag der Anmeldung: 09.03.2005
(73) Patentinhaber: Evonik Degussa GmbH, 45128 Essen (DE)
(72) Erfinder: GRASS, Michael, 45721 Haltern am See (DE); KAIZIK, Alfred, 45772 Marl (DE); BÜSCHKEN, Wilfried, 45721 Haltern am See (DE); TUCHLENSKI, Axel, 69469 Weinheim (DE); MASCHMEYER, Dietrich, 45657 Recklinghausen (DE); GAUDSCHUN, Kurt-Alfred, 45665 Recklinghausen (DE); BROCKSIEN, Frank, 48249 Dülmen (DE)
(86) Internationale Anmeldenummer: PCT/EP2003/004386
(87) Internationale Veröffentlichungsnummer: WO 2003/103830

(56) Entgegenhaltungen:
- EP-A- 0 067 058
- EP-A- 0 324 984
- WO-A-00/78704
- WO-A-98/57913
- WO-A-99/32427
- DE-A- 2 909 663
- DE-A- 10 054 347
- US-A- 3 636 108
- US-A- 5 202 475
- US-A- 5 578 546

## Beschreibung

Die Erfindung betrifft die Hydrierung von aromatischen Verbindungen, insbesondere die Herstellung von alicyclischen Polycarbonsäure estern durch Kernhydrierung der entsprechenden aromatischen Polycarbonsäure ester.

Alicyclische Polycarbonsäureester, wie beispielsweise die Ester der Cyclohexan-1,2-dicarbonsäure, werden als Schmierölkomponente und als Hilfsmittel bei der Metallverarbeitung eingesetzt. Weiterhin finden sie als Weichmacher für Polyolefine und für PVC Verwendung.

Für die Weichmachung von PVC werden zur Zeit überwiegend Ester der Phthalsäure, wie beispielweise Dibutyl-, Dioctyl-, Dinonyl- oder Didecylester, verwendet. Da über die Verwendung dieser Phthalate in letzter Zeit zunehmend kontrovers diskutiert wird, muss befürchtet werden, dass deren Einsatz in Kunststoffen eingeschränkt werden könnte. Alicyclische Polycarbonsäureester, von denen einige in der Literatur bereits als Weichmacher für Kunststoffe beschrieben sind, könnten dann als geeignete Ersatzstoffe zur Verfügung stehen.

In den meisten Fällen ist der wirtschaftlichste Weg zur Herstellung von alicyclischen Polycarbonsäureestern die Kernhydrierung der entsprechenden aromatischen Polycarbonsäureester, beispielsweise der o. g. Phthalate. Es sind hierzu bereits einige Verfahren bekannt:
In US 5 286 898 und US 5 319 129 werden Verfahren beschrieben, mit denen Dimethylterephthalat an geträgerten Pd-Katalysatoren, die mit Ni, Pt und/oder Ru dotiert sind, bei Temperaturen größer oder gleich 140 °C und einem Druck zwischen 50 und 170 bar zum entsprechenden Hexahydrodimethylterephthalat hydriert werden kann.

US 3 027 398 offenbart die Hydrierung von Dimethylterephthalat an geträgerten Ru-Katalysatoren bei 110 bis 140°C und 35 bis 105 bar.

In DE 28 23 165 werden aromatische Carbonsäureester an geträgerten Ni, Ru, Rh und/oder Pd-Katalysatoren zu den entsprechenden alicyclischen Carbonsäureestern bei 70 bis 250 °C und 30 bis 200 bar hydriert. Dabei wird ein makroporöser Träger mit einer mittleren Porengröße von 70 nm und einer BET-Oberfläche von ca. 30 m²/g eingesetzt.

In WO 99/32427 und WO 00/78704 werden Verfahren zur Hydrierung von Benzolpolycarbonsäureestem zu den entsprechenden alicyclischen Verbindungen offengelegt. Dabei werden Trägerkatalysatoren eingesetzt, die ein Metall der VIII. Nebengruppe alleine oder zusammen mit mindestens einem Metall der I. oder VII. Nebengruppe des Periodensystems enthalten und Makroporen aufweisen. Als bevorzugtes Metall der VIII. Nebengruppe wird Ruthenium eingesetzt. Zur Hydrierung werden drei verschiedene Katalysatortypen eingesetzt, die sich im Wesentlichen durch ihre mittleren Porendurchmesser und ihre BET-Oberflächen unterscheiden.

| | |
|---|---|
| Katalysator I: | mittlerer Porendurchmesser größer 50 nm und BET-Oberfläche kleiner 30 m²/g |
| Katalysator II: | mittlerer Porendurchmesser 5 bis 20 nm und BET-Oberfläche größer 50 m²/g |
| Katalysator III: | mittlerer Porendurchmesser größer 100 nm und BET-Oberfläche kleiner 15 m²/g |

Die für die Kernhydrierung von aromatischen Carbonsäuren oder deren Ester eingesetzten Katalysatoren sollen eine hohe Reaktionsgeschwindigkeit ermöglichen, nur einen geringen Anteil an Nebenprodukten generieren und eine lange Standzeit aufweisen.

Die Aktivität und Selektivität von Hydrierkatalysatoren hängt von deren Oberflächeneigenschaften wie Porengröße, BET-Oberfläche oder Oberflächenkonzentration der aktiven Metalle ab.

Nun ist ein Katalysator in einem kontinuierlich betriebenen Verfahren mechanischen, thermischen und chemischen Belastungen ausgesetzt, die die Porengröße bzw. die BET-Oberfläche verändern und damit die Aktivität und Selektivität dieses Katalysators herabsetzen.

So ist an vielen Katalysatoren neben der mechanischen Abrasion ein Vergrößern der Porenvolumina und Durchmesser durch Säurefraß zu beobachten.

Aromatische Polycarbonsäureester enthalten häufig geringe Mengen an Carbonsäuren, zusätzlich entstehen während der Kernhydrierung von Estern Säurespuren. Partialester von Polycarbonsäuren oder Polycarbonsäuren als solche sind aufgrund ihrer Struktur acide. Daher sollte ein für ein kontinuierliches Verfahren geeigneter Hydrierkatalysator auch bei höheren Temperaturen unter den Hydrierbedingungen gegen Säure beständig sein.

Die bekannten Katalysatoren erfüllen in Bezug auf die Aktivität, Selektivität oder Stabilität noch nicht die gewünschten Anforderungen. So ist beispielsweise bekannt, dass γ-Al₂-O₃ im Unterschied zu α-Al₂-O₃ nicht ausreichend säurestabil ist.

Es bestand daher die Aufgabe, die Katalysatoren für die Kernhydrierung von aromatischen Carbonsäuren und/oder deren Estern mit verbesserten Eigenschaftsprofilen zu entwickeln.

Es wurde nun überraschend gefunden, dass Katalysatoren, die mindestens ein Metall der achten Nebengruppe enthalten und aus einem Trägermaterial mit einem mittleren Porendurchmesser von 25 bis 50 nm und einer spezifischen Oberfläche von größer 30 m²/g bestehen, aromatische Carbonsäuren und/oder deren Ester (Voll- oder Partialester) in hoher Selektivität und Raum-Zeit-Ausbeute ohne nennenswerte Nebenreaktionen zu den entsprechenden alicyclischen Polycarbonsäuren oder deren Ester hydrieren.

Offenbart wird ein Katalysator zur Hydrierung aromatischer Verbindungen zu den entsprechenden alicyclischen Verbindungen, der mindestens ein Metall der achten Nebengruppe des Periodensystems auf oder in einem Trägermaterial enthält, wobei das Trägermaterial einen mittleren Porendurchmesser von 25 bis 50 nm und eine spezifische Oberfläche größer 30 m²/g aufweist.

Katalysatoren dieser Art können besonders zur Hydrierung von aromatischen Verbindungen verwendet werden.

Gegenstand der vorliegenden Erfindung ein Verfahren zur katalytischen Hydrierung von Alkyl-, Cycloalkyl- sowie Alkoxyalkylestern von aromatischen Polycarbonsäuren zu den entsprechenden alicyclischen Polycarbonsäureverbindungen, wobei die Alkoholkomponenten der Ester Alkoxyalkyl-, Cycloalkyl-, und/oder Alkylgruppen mit 3 bis 15 Kohlenstoffatomen, verzweigt oder unverzweigt, jeweils gleich oder unterschiedlich sind, mit Wasserstoff-haltigen Gasen an einem Katalysator, der mindestens ein Metall der achten Nebengruppe des Periodensystems auf oder in einem Trägermaterial enthält, wobei das Trägermaterial einen mittleren Porendurchmesser von 25 bis 50 nm und eine spezifische Oberfläche größer 30 m²/g aufweist und über 90 % des Gesamtporenvolumens der Trägermaterialien auf Meso- und Mikroporen mit einem Durchmesser zwischen 0.1 und 50 nm entfällt.

Die Katalysatoren können prinzipiell alle Metalle der achten Nebengruppe des Periodensystems enthalten. Vorzugsweise werden als Aktivmetalle Platin, Rhodium, Palladium, Kobalt, Nickel oder Ruthenium oder ein Gemisch aus zwei oder mehreren davon eingesetzt, wobei insbesondere Ruthenium als Aktivmetall eingesetzt wird.

Neben den bereits genannten Metallen können zusätzlich mindestens ein Metall der ersten und/oder siebten Nebengruppe des Periodensystems der Elemente in den Katalysatoren enthalten sein. Bevorzugt wird Rhenium und/oder Kupfer eingesetzt.

Der Gehalt der Aktivmetalle, d. h. der Metalle der ersten und/oder siebten und/oder achten Nebengruppe beträgt im Allgemeinen 0,1 bis 30 Massen-%. Der Edelmetallgehalt, d. h. der Metalle der achten Nebengruppe (genauer: der fünften und sechsten Periode), berechnet als Metall liegt im Bereich von 0,1 bis 10 Massen-%, insbesondere im Bereich von 0,8 bis 5 Massen-%, ganz besonders zwischen 1 und 3 Massen-%.

Zur Herstellung der Katalysatoren werden Trägermaterialien mit einem mittleren Porendurchmesser, der im Bereich von 25 bis 50 nm liegt, verwendet. (Die Bestimmung des mittleren Porendurchmesser erfolgt durch Hg-Porosimetrie, insbesondere nach DIN 66133.)

Bei den eingesetzten Trägermaterialien kann zwischen Mikroporen (Porendurchmesser kleiner 2 nm), Mesoporen (Porendurchmesser 2 bis 50 nm) und Makroporen (Porendurchmesser größer 50 nm) unterschieden werden. So sind hinsichtlich der Porenart Trägermaterialien mit folgenden Porenkombinationen einsetzbar.
a) nur Mesoporen
b) Mikroporen und Mesoporen
c) Mesoporen und Makroporen
d) Mikroporen und Mesoporen und Makroporen
e) Mikroporen und Makroporen.

Entscheidend für die Herstellung der Katalysatoren ist, dass unabhängig von der Porengrößenverteilung der mittlere Porendurchmesser des Trägermaterials zwischen 25 und 50 nm liegt. Vorzugsweise beträgt der mittlere Porendurchmesser 25 bis 40 nm, ganz besonders bevorzugt 30 bis 40 nm.

Daher können auch Trägermaterialien mit hohem Makroporenanteil (> 550 %) eingesetzt werden, sofern der mittlere Porendurchmesser zwischen 25 und 50 nm, vorzugsweise zwischen 25 und 40 nm, ganz besonders bevorzugt zwischen 30 und 40 nm beträgt.

Die spezifische Oberfläche des Trägers (-bestimmt nach dem BET-Verfahren durch Stickstoff-Adsorption, gemäß DIN 66131) ist größer als 30 m²/g, bevorzugt beträgt die spezifische Oberfläche 30 bis 90 m²/g bzw. 35 bis 90 m²/g, insbesondere zwischen 40 bis 60 m²/g.

Zur Herstellung der Katalysatoren werden Trägermaterialien verwendet, bei denen über 90 %, insbesondere über 95 % des Gesamtporenvolumens auf Mikro- und Mesoporen, d. h. Poren mit einem Durchmesser zwischen 0.1 und 50 nm, bevorzugt zwischen 0.1 und 20 nm, entfällt.

Als Träger für die Herstellung der Katalysatoren werden Feststoffe eingesetzt, deren mittlere Porendurchmesser und deren spezifische Oberfläche in den oben genannten Bereichen liegen. Als Träger können beispielsweise folgende Stoffe verwendet werden: Aktivkohle, Siliciumcarbid, Aluminiumoxid, Siliciumoxid, Alumosilikat, Titandioxid, Zirkoniumdioxid, Magnesiumoxid und/oder Zinkoxid oder deren Gemische.

Bevorzugt werden zur Herstellung der Katalysatoren Träger verwendet, die unter Hydrierbedingungen gegen Carbonsäuren resistent sind. Dies sind beispielsweise Aktivkohle, Siliciumcarbid, Siliciumdioxid, Titandioxid und/oder Zirkondioxid bzw. Gemische aus diesen Verbindungen.

Ganz besonders bevorzugt werden Titandioxide als Trägermaterialien verwendet. Titandioxid tritt in drei Modifikationen auf (Anatas, Rutil, Brookit) auf, von denen Anatas und Rutil die häufigsten sind. Für die Herstellung der erfindungsgemäßen Katalysatoren können alle Titandioxid-Modifikationen, Titandioxide, bei denen mindestens zwei Modifikationen nebeneinander vorliegen, oder Gemische unterschiedlicher Titandioxide verwendet werden, wenn sie hinsichtlich des mittleren Porendurchmessers und spezifischer Oberfläche im erfindungsgemäßen Bereich liegen. Ein bevorzugtes Trägermaterial ist Aerolyst 7711® (Verkaufsprodukt der Degussa AG, Düsseldorf). Dieser Träger besteht zu 15- 20 Massen-% aus Rutil und zu 80- 85 Massen-% aus Anatas. Weitere Titandioxidträger, die sich zur Herstellung der Katalysatoren eignen, sind beispielsweise Träger, die auf Basis von Titanoxiden aus einem Schwefelsäure-Verfahren hergestellt werden. Sie enthalten in der Regel > 98 % Anatas.

Die Katalysatoren können durch Auftragen mindestens eines Metalls der achten Nebengruppe des Periodensystems und gegebenenfalls mindestens eines Metalls der ersten und/oder siebten Nebengruppe des Periodensystems auf einem geeigneten Träger erhalten werden. Es ist auch möglich, die Aktivmetalle und den Träger gleichzeitig herzustellen, d. h. einen Vollkatalysator einzusetzen.

Die Auftragung kann durch Tränken des Trägers in wässrigen Metallsalzlösungen, wie z. B. wässrigen Rutheniumsalzlösungen, durch Aufsprühen entsprechender Metallsalzlösungen auf den Träger oder durch andere geeignete Verfahren erreicht werden. Als Metallsalze der ersten, siebten oder achten Nebengruppe des Periodensystems eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, wobei die Nitrate und Nitrosylnitrate bevorzugt sind.

Bei Katalysatoren, die neben dem Metall der achten Nebengruppe des Periodensystems noch weitere Metalle als Aktivmetall aufgetragen enthalten, können die Metallsalze bzw. Metallsalzlösungen gleichzeitig oder nacheinander aufgebracht werden.

Die mit Metallsalzlösung beschichteten bzw. getränkten Träger werden anschließend, vorzugsweise bei Temperaturen von 80 bis 150 °C, getrocknet und wahlweise bei Temperaturen von 200 bis 600°C calciniert. Bei getrennter Auftränkung wird der Katalysator nach jedem Tränkschritt getrocknet und wahlweise calciniert, wie oben beschrieben. Die Reihenfolge, in der Aktivkomponenten aufgebracht werden, ist dabei frei wählbar.

Optional kann die Aufbringung der Aktivkomponente(n), Trocknung und Calcinierung in einem Arbeitsgang erfolgen, beispielsweise durch Aufsprühen einer wässrigen Metallsalzlösung auf den Träger bei Temperaturen über 200°C.

Die Katalysatoren werden zweckmäßig in eine Form gebracht, die bei der Hydrierung einen geringen Strömungswiderstand bietet, wie beispielsweise Tabletten, Zylinder, Strangextrudate oder Ringe. Die Formgebung kann dabei wahlweise an verschiedenen Stellen der Katalysatorherstellung erfolgen.

Im erfindungsgemäßen Verfahren wird die Hydrierung in flüssiger Phase oder in der Gasphase durchgeführt. Die Hydrierung kann an suspendierten oder stückigen im Festbett angeordneten Katalysatoren kontinuierlich oder diskontinuierlich durchgeführt werden. Im erfindungsgemäßen Verfahren wird eine kontinuierliche Hydrierung an einem im Festbett angeordnetem Katalysator, bei dem sich unter Reaktionsbedingungen die Produkt/Eduktphase hauptsächlich im flüssigen Zustand befindet, bevorzugt.

Wenn die Hydrierung kontinuierlich an einem im Festbett angeordneten Katalysator durchgeführt wird, ist es zweckmäßig, den Katalysator vor der Hydrierung in die aktive Form zu überführen. Dies kann durch Reduktion des Katalysators mit Wasserstoff-haltigen Gasen nach einem Temperaturprogramm erfolgen. Dabei kann die Reduktion gegebenenfalls in Gegenwart einer flüssigen Phase, die über den Katalysator rieselt, durchgeführt werden. Als flüssige Phase kann dabei ein Lösemittel oder das Hydrierprodukt eingesetzt werden.

Für das erfindungsgemäße Verfahren können unterschiedliche Verfahrensvarianten gewählt werden. Es kann adiabatisch, polytrop oder praktisch isotherm, d. h. mit einem Temperaturanstieg von typischerweise kleiner als 10 °C, ein- oder mehrstufig durchgeführt werden. Im letzteren Falle kann man alle Reaktoren, zweckmäßig Rohrreaktoren, adiabatisch oder praktisch isotherm sowie einen oder mehrere adiabatisch und die anderen praktisch isotherm betreiben. Weiterhin ist es möglich, die aromatischen Verbindungen im geraden Durchgang oder unter Produktrückführung zu hydrieren.

Das erfindungsgemäße Verfahren wird bevorzugt in der Flüssig/Gas-Mischphase oder Flüssigphase in Dreiphasenreaktoren im Gleichstrom durchgeführt, wobei das Hydriergas in an sich bekannter Weise im flüssigen Edukt/Produktstrom verteilt wird. Im Interesse einer gleichmäßigen Flüssigkeitsverteilung, einer verbesserten Reaktionswärmeabfuhr und einer hohen Raum-Zeit-Ausbeute werden die Reaktoren vorzugsweise mit hohen Flüssigkeitsbelastungen von 15 bis 120, insbesondere von 25 bis 80 m³ pro m² Querschnitt des leeren Reaktors und Stunde betrieben. Wird ein Reaktor im geraden Durchgang betrieben, so kann die spezifische Katalysatorbelastung (LHSV) Werte zwischen 0,1 und 10 h⁻¹ annehmen.

Die Hydrierung kann in Ab- oder vorzugsweise in Anwesenheit eines Lösungsmittels durchgeführt werden. Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten.

Beispielsweise können folgende Stoffe als Lösemittel eingesetzt werden:
Geradkettige oder cyclische Ether, wie beispielsweise Tetrahydrofuran oder Dioxan, sowie aliphatische Alkohole, in denen der Alkylrest 1 bis 13 Kohlenstoffatome aufweist.

Bevorzugt verwendbare Alkohole sind Isopropanol, n-Butanol, Isobutanol, n-Pentanol, 2-Ethylhexanol, Nonanole, technische Nonanolgemische, Decanol, technische Decanolgemische, Tridecanole.

Bei Einsatz von Alkoholen als Lösemittel kann es zweckmäßig sein, denjenigen Alkohol oder dasjenige Alkoholgemisch zu verwenden, das bei der Verseifung des Produkts entstehen würde. Dadurch würde die Nebenproduktbildung durch Umesterung ausgeschlossen. Ein weiteres bevorzugtes Lösemittel ist das Hydrierprodukt selbst.

Durch die Verwendung eines Lösemittels kann die Aromatenkonzentration im Reaktorzulauf begrenzt werden, wodurch eine bessere Temperaturkontrolle im Reaktor erreicht werden kann. Dies kann eine Minimierung von Nebenreaktionen und somit eine Erhöhung der Produktausbeute zur Folge haben. Bevorzugt liegt der Aromatengehalt im Reaktorzulauf zwischen 1 und 35 %, insbesondere zwischen 5 und 25 %. Der gewünschte Konzentrationsbereich kann bei Reaktoren, die in Schlaufenfahrweise betrieben werden, durch das Kreislaufverhältnis (Mengenverhältnis von rückgeführten Hydrieraustrag zu Edukt) eingestellt werden.

Das erfindungsgemäße Verfahren wird in einem Druckbereich von 3 bis 300 bar, insbesondere zwischen 15 und 200 bar, ganz besonders zwischen 50 und 200 bar durchgeführt. Die Hydriertemperaturen liegen zwischen 50 und 250, insbesondere zwischen 100 und 200 °C.

Als Hydriergase können beliebige Wasserstoff-haltige Gasgemische, die keine schädlichen Mengen an Katalysatorgiften wie beispielsweise Kohlenmonoxid oder Schwefelwasserstoff enthalten, eingesetzt werden. Die Verwendung von Inertgasen ist optional, bevorzugt wird Wasserstoff in einer Reinheit von größer 95 %, insbesondere größer 98 % eingesetzt. Inertgasbestandteile können beispielsweise Stickstoff oder Methan sein.

Die einzelnen Reaktoren können mit Frisch-Wasserstoff beschickt werden. Um den Wasserstoffverbrauch und die mit dem Abgas bedingten Austragsverluste zu minimieren, ist es jedoch zweckmäßig, das Abgas eines Reaktors als Hydriergas eines anderen Reaktors zu verwenden. Beispielsweise ist es bei einem Verfahren, das in zwei hintereinander geschalteten Reaktoren durchgeführt wird, vorteilhaft, Frisch-Wasserstoff in den zweiten Reaktor einzuspeisen und das Abgas des zweiten Reaktors in den ersten Reaktor zu leiten. In diesem Falle strömen Einsatzstoff und Hydriergas in entgegengesetzter Reihenfolge durch die Reaktoren. Es ist zweckmäßig, den Wasserstoffüberschuss, bezogen auf die stöchiometrisch notwendige Menge, unter 30 %, insbesondere unter 10 %, ganz besonders unter 5 % zu halten.

Werden Nonylphthalate oder Gemische von Nonylphthalate zu den entsprechenden 1,2-Cyclohexandicarbonsäureestern umgesetzt, so wird die Hydrierung vorzugsweise in der Flüssig/Gas-Mischphase oder Flüssigphase in zwei hintereinandergeschalteten Reaktoren durchgeführt. Dabei wird der erste Reaktor in Schlaufenfahrweise betrieben, d. h. ein Teil des Hydrieraustrags des ersten Reaktors wird zusammen mit Frisch-Edukt auf den Kopf des ersten Reaktors geleitet. Der andere Teil des Austrags des ersten Reaktors wird in einem zweiten Reaktor im geraden Durchgang hydriert. Es ist auch möglich, anstelle eines großen Schlaufenreaktors mehrere kleinere Schlaufenreaktoren, die in Reihe oder parallel angeordnet sind, zu verwenden. Ebenso ist es möglich, anstatt eines großen Reaktors, der im geraden Durchgang durchströmt wird, mehrere Reaktoren, die in Reihe oder parallel miteinander verschaltet sind, zu betreiben. Bevorzugt werden jedoch nur ein Schlaufenreakor und nur ein Reaktor, der im geraden Durchgang betrieben wird, verwendet.

Das erfindungsgemäße Verfahren wird vorzugsweise unter folgenden Bedingungen durchgeführt:
Im Zulauf des ersten Reaktors (Schlaufenreaktor) liegt die Konzentration des Edukts zwischen 5 und 30 Massen-%, insbesondere zwischen 8 und 15 Massen%.

Im Hydrieraustrag des ersten Reaktors liegt die Konzentration des Edukts zwischen 0,3 und 8 Massen-%, insbesondere zwischen 1,5 und 4 Massen-%.

Die spezifische Katalysatorbelastung (LHSV, Liter Frisch-Edukt je Liter Katalysator je Stunde) im Schlaufenreaktor beträgt 0,1 bis 5, h⁻¹ insbesondere 0,5 bis 3 h⁻¹.

Die Oberflächenbelastung im Schlaufenreaktor liegt im Bereich von 25 bis 140 m³/m²/h, insbesondere im Bereich von 50 bis 90 m³/m²/h.

Die durchschnittlichen Hydriertemperaturen im Schlaufenreaktor sind 70 bis 150 °C, insbesondere 80 bis 120 °C.

Der Hydrierdruck im Schlaufenreaktor beträgt 25 bis 200 bar, insbesondere 80 bis 110 bar.

Im Edukt des zweiten Reaktors ist die Konzentration an Edukt kleiner als 0,3 Massen-%, insbesondere kleiner als 0,1 Massen-%, ganz besonders kleiner als 0,05 Massen-%.

Die spezifische Katalysatorbelastung im zweiten Reaktor (Liter Nonylphthalat je Liter Katalysator je Stunde) beträgt 1 bis 8 h⁻¹, insbesondere 2 bis 5 h⁻¹.

Im zweiten Reaktor liegt die durchschnittliche Temperatur zwischen 70 und 150 °C, insbesondere 80 und 120 °C.

Der Hydrierdruck im zweiten Reaktor beträgt 25 bis 200 bar, insbesondere 80 bis 100 bar.

Die Verfahrensvarianten eigenen sich insbesondere zur Hydrierung von Phthalsäureestern, besonders für Nonylphthalate (als Isomerengemisch "Isononylphthalat" z. B. VESTINOL 9 der OXENO GmbH).

Nach dem erfindungsgemäßen Verfahren werden Alkyl-, Cycloalkyl- sowie Alkoxyalkylester von aromatischen Polycarbonsäuren zu den entsprechenden alicyclischen Polycarbonsäureverbindungen umgesetzt. Dabei können sowohl Vollester als auch Partialester hydriert werden. Unter Vollester wird eine Verbindung verstanden, bei der alle Säuregruppen verestert sind. Partialester sind Verbindungen mit mindestens einer freien Säuregruppe (oder ggf. einer Anhydridgruppe) und mindestens einer Estergruppe.

Werden im erfindungsgemäßen Verfahren Polycarbonsäureester eingesetzt, so enthalten diese bevorzugt 2, 3 oder 4 Esterfunktionen.

Die im erfindungsgemäßen Verfahren verwendeten aromatischen Polycarbonsäureester sind bevorzugt Benzol-, Diphenyl-, Naphthalin- und/oder Anthracenpolycarbonsäure ester wie z. B. Alkylester mit 2 bis 15 Kohlenstoffatomen. Die so erhaltenen alicyclischen Polycarbonsäuren bzw. deren Derivate bestehen aus einem oder mehreren, ggf. durch eine C-C-Bindung verknüpfte oder ankondensierte C₆-Ringe.

Die Alkoholkomponente der aromatischen Carbonsäureester besteht bevorzugt aus verzweigten oder unverzweigten Alkyl-, Cycloalkyl- oder Alkoxyalkylgruppen mit 1 - 25 Kohlenstoffatomen. Diese können in einem Molekül eines Polycarbonsäureesters gleich oder unterschiedlich sein, d.-h. sie können gleiche oder verschiedene Isomeren oder Kettenlängen besitzen. Selbstverständlich können auch Isomere bezüglich des Substitutionsmusters des aromatischen Systems in Form eines Gemisches eingesetzt werden, z. B. ein Gemisch aus Phthalsäureester und Terephthalsäureester.

In einer bevorzugten Ausführungsform betrifft die vorliegende Erfindung ein Verfahren zur Hydrierung der 1,2-; 1,3- oder 1,4-Benzoldicarbonsäureester, und/oder der 1,2,3-; 1,2,4- oder 1,3,5-Benzoltricarbonsäureester, d. h. es werden die Isomere der 1,2-; 1,3- oder 1,4-Cyclohexandicarbonsäureester, oder der 1,2,3-; 1,3,5- oder 1,2,4-Cyclohexantricarbonsäureester erhalten.

Im erfindungsgemäßen Verfahren können beispielsweise Ester folgender aromatischer Carbonsäuren eingesetzt werden: 1,2-Naphthalindicarbonsäure, 1,3-Naphthalindicarbonsäure, 1,4-Naphthalindicarbonsäure, 1,5-Naphthalindicarbonsäure, 1,6-Naphthalindicarbonsäure, 1,7-Naphthalindicarbonsäure, 1,8-Naphthalindicarbonsäure, Phthalsäure (Benzol-1,2-dicarbonsäure), Isophthalsäure (Benzol-1,3-dicarbonsäure), Terephthalsäure (Benzol-1,4-dicarbonsäure), Benzol-1,2,3-tricarbonsäure, Benzol-1,2,4-tricarbonsäure (Trimellitsäure), Benzol-1,3,5-tricarbonsäure (Trimesinsäure), Benzol-1,2,3,4-tetracarbonsäure. Weiterhin können Säuren eingesetzt werden, die aus den genannten Säuren durch Substitution eines oder mehrerer am aromatischen Kern gebundenen Wasserstoffatome durch Alkyl-, Cycloalkyl- oder Alkoxyalkylgruppen entstehen.

Es ist möglich, Alkyl-, Cycloalkyl- sowie Alkoxyalkylester z. B. der oben genannten Säuren zu verwenden, wobei diese Reste unabhängig von einander 1 bis 25, insbesondere 3 bis 15, ganz besonders 8 bis 13 C-Atome, insbesondere 9 C-Atome, umfassen. Diese Reste können linear oder verzweigt sein. Hat ein Einsatzprodukt mehr als eine Estergruppe, dann können diese Reste gleich oder verschieden sein.

Im erfindungsgemäßen Verfahren können als Ester einer aromatischen Polycarbonsäure beispielsweise folgende Verbindungen eingesetzt werden: Terephthalsäuremonomethylester, Terephthalsäuredimethylester, Terephthalsäurediethylester, Terephthalsäuredi-n-propylester, Terephthalsäuredibutylester, Terephthalsäurediisobutylester, Terephthalsäuredi-tert.-butylester, Terephthalsäuremonoglykolester, Terephthalsäurediglykolester, Terephthalsäure-n-octylester, Terephthalsäurediisooctylester, Terephthalsäuredi-2-ethylhexylester, Terephthalsäuredi-n-nonylester, Terephthalsäurediisononylester, Terephthalsäuredi-n-decylester, Terephthalsäuredi-n-undecylester, Terephthalsäurediisodecylester, Terephthalsäurediisododecylester, Terephthalsäure-ditridecylester, Terephthalsäuredi-n-octadecylester, Terephthalsäurediisooctadecylester, Terephthalsäuredi-n-eicosylester, Terephthalsäuremonocyclohexylester; Phthalsäuremonomethylester, Phthalsäuredimethylester, Phthalsäuredi-n-propylester, Phthalsäuredi-n-butylester, Phthalsäurediisobutylester, Phthalsäuredi-tert. -butylester, Phthalsäuremonoglykolester, Phthalsäurediglykolester, Phthalsäuredi-n-octylester, Phthalsäurediisooctylester, Phthalsäuredi-2-ethylhexylester, Phthalsäuredi-n-nonylester, Phthalsäurediisononylester, Phthalsäuredi-n-decylester, Phthalsäuredi-2-propylheptylester, Phthalsäurediisodecylester, Phthalsäuredi-n-undecylester, Phthalsäurediisoundecylester, Phthalsäureditridecylester, Phthalsäuredi-n-octadecylester, Phthalsäurediisooctadecylester, Phthalsäuredi-n-eicosylester, Phthalsäuremonocyclohexylester; Phthalsäuredicyclohexylester, Isophthalsäuremonomethylester, Isophthalsäuredimethylester, Isophthalsäuredimethylester, Isophthalsäurediethylester, Isophthalsäuredi-n-propylester, Isophthalsäuredi-n-butylester, Isophthalsäurediisobutylester, Isophthalsäuredi-tert.-butylester, Isophthalsäuremonoglykolester. Isophthalsäurediglykolester, Isophthalsäuredi-n-octylester, Isophthalsäurediisooctylester, Isophthalsäuredi-2-ethylhexylester, Isophthalsäuredi-n-nonylester, Isophthalsäurediisononylester, Isophthalsäuredi-n-decylester, Isophthalsäurediisodecylester, Isophthalsäuredi-n-undecylester, Isophthalsäuredi-isododecylester, Isophthalsäuredi-n-dodecylester, Isophthalsäureditridecylester, Isophthalsäuredi-n-octadecylester, Isophthalsäurediisooctadecylester, Isophthalsäuredi-n-eicosylester, Isophthalsäuremonocyclohexylester.

Das erfindungsgemäße Verfahren ist prinzipiell auch auf Benzoesäure ester anwendbar. Hierunter werden neben Benzoesäurealkylester auch Benzoate von Diolen, wie beispielsweise Glycoldibenzoat, Diethylenglycolbenzoat, Triethylenglycoldibenzoat oder Propylenglycoldibenzoat, verstanden. Die Alkoholkomponente der Benzoesäurealkylester kann aus 1 bis 25, bevorzugt 8 bis 13 Kohlenstoffatomen, jeweils linear oder verzweigt, bestehen.

Es können auch Gemische aus zwei oder mehreren Polycarbonsäureestern eingesetzt werden. Solche Gemische können beispielsweise auf folgenden Wegen erhalten werden:
a) Eine Polycarbonsäure wird mit einem Alkohol derart partiell verestert, dass Voll- und Partialester nebeneinander vorliegen.
b) Ein Gemisch von mindestens zwei Polycarbonsäuren wird mit einem Alkohol verestert, wobei ein Gemisch von mindestens zwei Vollestern entsteht.
c) Eine Polycarbonsäure wird mit einem Alkoholgemisch versetzt, wobei ein entsprechendes Gemisch der Vollester entstehen kann.
d) Eine Polycarbonsäure wird mit einem Alkoholgemisch partiell verestert.
e) Ein Gemisch von mindestens zwei Carbonsäuren wird mit einem Alkoholgemisch partiell verestert.
f) Ein Gemisch aus mindestens zwei Polycarbonsäuren wird mit einem Alkoholgemisch partiell verestert.
Bei den Umsetzungen a) bis f) können an Stelle der Polycarbonsäuren auch die entsprechenden Anhydride eingesetzt werden.

Großtechnisch werden aromatische Ester, insbesondere die Vollester auf Weg c) häufig aus Alkoholgemischen hergestellt.

Entsprechende Alkoholgemische sind beispielsweise:
C₅-Alkoholgemische, hergestellt aus linearen Butenen durch Hydroformylierung und anschließender Hydrierung;
C₅-Alkoholgemische, hergestellt aus Butengemischen, die lineare Butene und Isobuten enthalten, durch Hydroformylierung und anschließende Hydrierung;
C₆-Alkoholgemische, hergestellt aus einem Penten oder aus einem Gemisch von zwei oder mehreren Pentenen, durch Hydroformylierung und anschließende Hydrierung;
C₇-Alkoholgemische, hergestellt aus Triethylen oder Dipropen oder einem Hexenisomer oder einem sonstigen Gemisch von Hexenisomeren, durch Hydroformylierung und anschließende Hydrierung;
C₈-Alkoholgemische, wie 2-Ethylhexanol (2 Isomere), hergestellt durch Aldolkondensation von n-Butyraldehyd und anschließende Hydrierung;
C₉-Alkoholgemische, hergestellt aus C₄-Olefinen durch Dimerisierung, Hydroformylierung und Hydrierung. Dabei kann zur Herstellung der C₉-Alkohole von Isobuten oder von einem Gemisch linearer Butene oder von Gemischen mit linearen Butenen und Isobuten ausgegangen werden. Die C₄-Olefine können mit Hilfe unterschiedlicher Katalysatoren dimerisiert werden, wie beispielsweise Protonensäuren, Zeolithe, metallorganische Nickelverbindungen oder feste nickelhaltige Kontakte. Die Hydroformylierung der C₈-Olefingemische kann mit Hilfe von Rhodium- oder Kobaltkatalysatoren erfolgen. Es gibt daher eine Vielzahl von technischen C₉-Alkoholgemischen.
C₁₀-Alkoholgemische, hergestellt aus Tripropylen durch Hydroformylierung und anschließende Hydrierung; 2-Propylheptanol (2 Isomere), hergestellt durch Aldolkondensation von Valeraldehyd und anschließende Hydrierung;
C₁₀-Alkoholgemische, hergestellt aus einem Gemisch von mindestens zwei C₅-Aldehyden durch Aldolkondensation und anschließende Hydrierung;
C₁₃-Alkolgemische, hergestellt aus Hexaethylen, Tetrapropylen oder Tributen durch Hydroformylierung und anschließende Hydrierung.

Weitere Alkoholgemische können durch Hydroformylierung und anschließende Hydrierung aus Olefinen bzw. Olefingemischen gewonnen werden, die beispielsweise bei Fischer-Tropsch-Synthesen, bei Dehydrierungen von Kohlenwasserstoffen, Metathesereaktionen, beim Polygasverfahren oder anderen technischen Prozessen anfallen.

Darüber hinaus können auch Olefingemische mit Olefinen unterschiedlicher C-Zahlen für die Herstellung von Alkoholgemischen eingesetzt werden.

Im erfindungsgemäßen Verfahren können alle Estergemische, hergestellt aus aromatischen Polycarbonsäuren und den oben genannten Alkoholgemischen, eingesetzt werden. Erfindungsgemäß werden bevorzugt Ester, hergestellt aus Phthalsäure oder Phthalsäureanhydrid und einem Gemisch isomerer Alkohole mit 6 bis 13 C-Atomen, eingesetzt.

Beispiele für technische Phthalate, die im erfindungsgemäßen Verfahren eingesetzt werden können, sind folgende Produkte mit den Handelsnamen:
Vestinol C (Di-n-butylphthalat) (CAS Nr.84-74-2); Vestinol IB (Di-i-butylphthalat) (CAS Nr. 84-69-5); Jayflex DINP (CAS Nr.68515-48-0); Jayflex DIDP (CAS Nr.68515-49-1); Palatinol 9P (68515-45-7); Vestinol 9 (CAS Nr. 28553-12-0); TOTM (CAS Nr. 3319-31-1); Linplast 68-TM: Palatinol N (CAS Nr. 28553-12-0); Jayflex DHP (CAS Nr. 68515-50-4); Jayflex DIOP (CAS Nr. 27554-26-3); Jayflex UDP (CAS Nr. 68515-47-9); Jayflex DIUP (CAS Nr. 85507-79-5); Jayflex DTDP (CAS Nr.68515-47-9); Jayflex L9P (CAS Nr. 68515-45-7); Jayflex L911P (CAS Nr. 68515-43-5); Jayflex L11P (CAS Nr. 3648-20-2); Witamol 110 (CAS Nr. 68515-51-5); Witamol 118 (Di-n-C8-C10-alkylphthalat) (CAS Nr.71662-46-9); Unimoll BB (CAS Nr. 85-68-7); Linplast 1012 BP (CAS Nr. 90193-92-3); Linplast 13XP (CAS Nr.27253-26-5); Linplast 610P (CAS Nr. 68515-51-5); Linplast 68 FP (CAS Nr. 68648-93-1); Linplast 812 HP (CAS Nr. 70693-30-0); Palatinol AH (CAS Nr. 117-81-7); Palatinol 711 (CAS Nr. 68515-42-4); Palatinol 911 (CAS Nr. 68515-43-5); Palatinol 11 (CAS Nr. 3648-20-2); Palatinol Z (CAS Nr.26761-40-0); Palatinol DIPP (CAS Nr. 84777-06-0); Jayflex 77 (CAS Nr. 71888-89-6); Palatinol 10 P (CAS Nr. 533-54-0); Vestinol AH (CAS Nr. 117-81-7).

Es sei darauf hingewiesen, dass bei der Kernhydrierung aromatischer Polycarbonsäure ester aus jedem eingesetzten Isomer mindestens zwei stereoisomere Hydrierprodukte entstehen können. Die Mengenverhältnisse der dabei entstandenen Stereoisomeren zueinander hängen vom verwendeten Katalysator und von den Hydrierbedingungen ab.

Alle Hydrierprodukte mit beliebigen Verhältnis(sen) der Stereoisomeren zueinander können ohne Auftrennung verwendet werden.

Die Verwendung der erfindungsgemäß hergestellten alicyclischen Polycarbonsäureester als Weichmacher in Kunststoffen ist möglich. Bevorzugte Kunststoffe sind PVC, Homo- und Copolymere auf Basis von Ethylen, Propylen, Butadien, Vinylacetat, Glycidylacrylat, Glycidylmethacrylat, Acrylaten, Acrylaten mit am Sauerstoffatom der Estergruppe gebundenen Alkylresten von verzweigten oder unverzweigten Alkoholen mit einem bis zehn Kohlenstoffatome(n), Styrol, Acrylnitril, Homo- oder Copolymere von cyclischen Olefinen.

Als Vertreter der obigen Gruppen seien beispielsweise folgende Kunststoffe genannt: Polyacrylate mit gleichen oder verschiedenen Alkylresten mit 4 bis 8 C-Atomen, gebunden am Sauerstoffatom der Estergruppe, insbesondere mit dem n-Butyl-, n-Hexyl-, n-Octyl- und 2-Ethylhexylrest, und Isononylrest, Polymethacrylat, Polymethylmethacrylat, Methylacrylat-Butylacrylat-Copolymere, Methylmethacrylat-Butylmethacrylat-Copolymere, EthylenVinylacetat-Copolymere, chloriertes Polyethylen, Nitrilkautschuk, Acrylnitril-Butadien-StyrolCopolymere, Ethylen-Propylen-Copolymere, Ethylen-Propylen-Dien-Copolymere, Styrol-Acrylnitril-Copolymere, Acrylnitril-Butadien-Kautschuk, Styrol-Butadien-Elastomere, Methylmethacrylat-Styrol-Butadien-Copolymere und/oder Nitrocellulose.

Darüber hinaus können die erfindungsgemäß hergestellten alicyclischen Polycarbonsäureester zur Modifizierung von Kunststoffmischungen, beispielsweise der Mischung eines Polyolefins mit einem Polyamid, eingesetzt werden.

Offenbart werden Gemische aus Kunststoffen und den erfindungsgemäß hergestellten alicyclischen Polycarbonsäureestern. Geeignete Kunststoffe sind die bereits genannten Verbindungen. Solche Gemische enthalten bevorzugt mindestens 5 Massen-%, besonders bevorzugt 20-80 Massen-%, ganz besonders bevorzugt 30-70 Massen-% der alicyclischen Polycarbonsäureester.

Gemische aus Kunststoffen, insbesondere PVC, die einen oder mehrere der erfindungsgemäß hergestellten alicyclischen Polycarbonsäureester enthalten, können beispielsweise in folgenden Produkten enthalten sein, bzw. zu deren Herstellung verwendet werden:
Gehäuse für Elektrogeräte, wie beispielsweise Küchengeräte, Computergehäuse, Gehäuse und Bauteile von Phono- und Fernsehgeräte, Rohrleitungen, Apparate, Kabel, Drahtummantelungen, Isolierbänder, Fensterprofile, im Innenausbau, im Fahrzeug- und Möbelbau, Plastisole, in Bodenbelägen, medizinische Artikel, Lebensmittelverpackungen, Dichtungen, Folien, Verbundfolien, Schallplatten, Kunstleder, Spielzeug, Verpackungsbehälter, Klebebandfolien, Bekleidung, Beschichtungen, als Fasern für Gewebe.

Neben den obengenannten Anwendungen können die erfindungsgemäß hergestellten alicyclischen Polycarbonsäureester als Schmierölkomponente, als Bestandteil von Kühlflüssigkeiten und Metallbearbeitungsflüssigkeiten verwendet werden. Ebenso können sie als Komponente in Farben, Lacken, Tinten und Klebstoffen eingesetzt werden.

Die folgenden Beispiele sollen die Erfindung erläutern, ohne deren Anwendungsbreite, die sich aus der Beschreibung und den Patentansprüchen ergibt, einzuschränken.

### Beispiele

In den folgenden Beispielen wurden Katalysatoren eingesetzt, die aus den in der Tabelle 1 zusammen mit den für die Erfindung relevanten physikalischen Daten ausgelisteten Trägermaterialien hergestellt worden waren.

**Tabelle 1: Eigenschaften der verwendeten Träger**

| **Material** | **BET-Oberfläche in g/m² nach DIN 66131 (N₂**-**Adsorption)** | **mittlerer Porendurchmesser dp in nm nach DIN 66133 (Hg-Porosimetrie)** | **Gesamtporenvolumen in ml/g** | **Anteil des Porenvolumens der Makroporen in %** | **Anteil des Porenvolumens der Summe aus Meso- und Mikroporen in %** | **Hersteller oder Typenbezeichnung** |
|---|---|---|---|---|---|---|
| A: TiO₂ | 48 | 34,2 | 0,46 | <5 | > 95 | Degussa Aerolyst 7711 |
| B: ZrO₂ nicht erfindungsge | 56 | 25,1 | 0,29 | > 55 | < 45 | Degussa H0907 |
| mäß | | | | | | |
| C: α-Al₂-O nicht erfindungsge | 7 | 206,5 | 0,64 | > 97 | <3 | Axence SP 512 |
| mäß | | | | | | |

Das Gesamtporenvolumen wurde aus der Summe der Porenvolumina der Poren mit dp > 7,6 nm (bestimmt mit der Hg-Porosimetrie) und Poren mit dp < 7,6 nm (bestimmt mit der N2-Adsorptionsmethode) ermittelt.

### Herstellung der Hydrierkatalysatoren A, B und C

Für die Herstellung von Hydrierkatalysatoren auf der Basis der in Tabelle 1 aufgeführten Träger, wurden die Träger zuerst bei 80 °C getrocknet. Nach der Trocknung wurden die Träger mit einer wässrigen Ruthenium (III)-Nitrat-Lösung, die eine Konzentration von 0,8 Massen-% Ruthenium enthielt, getränkt oder sprühgetrocknet.

Für die Tränkung des Trägers wurde die salpetersaure Ru-Lösung mit Wasser auf ein dem Porenvolumen des Trägers entsprechendes Volumen verdünnt.
Das Aufbringen der Ru-Lösung auf das Trägermaterial erfolgte durch Auftropfen oder vorzugsweise durch gleichmäßiges Versprühen unter Umwälzung des Trägers. Nach der Trocknung bei 120 °C unter Stickstoff wurde der mit Ruthenium-Salz belegte Träger im Wasserstoff/Stickstoff-Gemisch (Verhältnis 1 : 9) bei 200 °C über 6 Stunden lang aktiviert (reduziert).

**Anmerkung:** Die so hergestellten Katalysatoren wurden im folgenden Text mit dem gleichen Großbuchstaben wie der zugrundeliegende Träger bezeichnet, wobei in einer nachgestellten Klammer das Aktivmetall und dessen Gehalt angegeben wurde.

### Hydrierbeispiele 1-5

Die Hydrierversuche wurden nach folgender allgemeinen Vorschrift durchgeführt:
90,7 g des Katalysators wurden in einem Katalysatorkörbchen vorgelegt, in einem 1000 ml-Druckreaktor sorgfältig nach obiger Vorschrift im Wasserstoffstrom reduziert und dann mit 590 g flüssigem Diisononylphthalat (Vestinol 9, OXENO Olefinchemie GmbH) versetzt. Die Hydrierung des DINP erfolgte mit reinem Wasserstoff. Nach Hydrierung des Einsatzstoffes wurde der Reaktor entspannt und das Reaktionsgemisch mittels Gaschromatographie auf seinen Gehalt am Zielprodukt Cyclohexan-1,2-dicarbonsäurediisononylester (DINCH) analysiert. Der Umsatz an DINP betrug danach stets < 99,9 %.

Die Versuchsbedingungen der Hydrierbeispiele und deren Ergebnisse wurden in Tabelle 2 zusammengefasst:

**Tabelle 2: DINP-Hydrierung / Hydrierbeispiele**

| Hydrier-beispiele | Katalysator | Edukt | Druck in bar | Temperatur in °C | Reaktionszeit in Stunden | Gehalt an DINCH in % |
|---|---|---|---|---|---|---|
| 1* | A (1 % Ru) | DINP | 200 | 80 | 3,5 | 99,4 |
| 2* | A (1 % Ru) | DINP | 200 | 120 | 1 | 99,2 |
| 3* | A (1 % Ru) | DINP | 50 | 120 | 2,5 | 99,3 |
| 4 | B (1 % Ru) | DINP | 200 | 120 | 2 | 99,5 |
| 5 | C (1 % Ru) | DINP | 200 | 80 | 20 | 99,4 |

| | | | | | | |
|---|---|---|---|---|---|---|
| * erfindungsgemäß | | | | | | |

### Beispiel 6: Hydrierung von Phthalsäuremonoisononylester (erfindungsgemäß)

444 g Phthalsäureanhydrid (3 mol) und 432 g Isononanol (3 mol) (Vorstufe von Vestinol 9) wurden in einem Rundkolben mit Innenthermometer, Rührer und aufgesetztem Rückflusskühler langsam erhitzt. Bei einer Temperatur von 117 °C begann die Monoesterbildung, was sich durch einen starken Temperaturanstieg bemerkbar machte. Sofort nach Ansteigen der Temperatur wurde die Wärmezufuhr unterbrochen. Nach etwa 10 Minuten wurde der Ansatz, der inzwischen seine Endtemperatur von etwa 150 °C erreicht hatte, abgekühlt. Gaschromatographisch ließ sich eine Zusammensetzung von etwa 95 Massen-% Monoester, 3 Massen-% Diester, 0,5 Massen-% Isononanol und 1,5 Massen-% Phthalsäure ermitteln.

487 g (1,67 mol bezogen auf reinen Monoester) dieses Gemisches wurden ohne weitere Aufarbeitung mit 240 g (1,67 mol) Isononanol (Vorstufe von Vestinol 9) gemischt und unter Stickstoffatmosphäre in einem 1000 ml-Reaktor eingefüllt. Nach Zugabe von 70,7 g des Katalysators A (1 % Ru) wurde bei 200 bar und 120 °C mit Wasserstoff hydriert. Nach Beendigung der Kernhydrierung der aromatischen Carbonsäurederivate wurde der Reaktor entspannt. Der Reaktoraustrag wurde in eine Standard-Veresterungsapparatur überführt, mit weiteren 120 g (0,83 mol) Isononanol und etwa 0,07 g Tetrabutyltitanat gemischt und unter Standardbedingungen zum Diisononylcyclohexan-1,2-dicarboxylat (DINCH) verestert.

Nach Abdestillieren des überschüssigen Alkohols, Neutralisation und Aufarbeitung des Rohprodukts durch Wasserdampfdestillation konnte DINCH in einer Reinheit von 99,4 % erhalten werden.

### Beispiel 7: Säurestabilität des Katalysators A

In Gegenwart von Katalysator A (1 % Ru) wurde eine gesättigte wässrige Lösung von Phthalsäure bei 100 °C und 100 bar zu 1,2-Cyclohexandicarbonäure hydriert. Nach Beendigung der Hydrierung wurde die Lösung abgelassen, und Reaktor sowie Katalysator mit Methanol, Isononanol und DINP gespült. Anschließend wurde wieder DINP unter zu Beispiel 2 analogen Bedingungen hydriert. Es wurde festgestellt, dass die Hydrierung bei gleichen Selektivitäten bei einer mindestens gleichen Aktivität ablief.

Wie aus Tabelle 2 ersichtlich, ist der Katalysator A den Katalysatoren B und C bezüglich ihrer Aktivität klar überlegen.

## Patentansprüche

1. Verfahren zur katalytischen Hydrierung von Alkyl-, Cycloalkyl- sowie Alkoxyalkylestern von aromatischen Polycarbonsäuren zu den entsprechenden alicyclischen Polycarbonsäureverbindungen, wobei die Alkoholkomponenten der Ester Alkoxyalkyl-, Cycloalkyl-, und/oder Alkylgruppen mit 3 bis 15 Kohlenstoffatomen, verzweigt oder unverzweigt, jeweils gleich oder unterschiedlich sind, mit Wasserstoff-haltigen Gasen an einem Katalysator, der mindestens ein Metall der achten Nebengruppe des Periodensystems auf oder in einem Trägermaterial enthält,
**dadurch gekennzeichnet,**
**dass** das Trägermaterial einen mittleren Porendurchmesser von 25 bis 50 nm und eine spezifische Oberfläche größer 30 m²/g aufweist und über 90 % des Gesamtporenvolumens der Trägermaterialien auf Meso- und Mikroporen mit einem Durchmesser zwischen 0.1 und 50 nm entfällt.

2. Verfahren nach Anspruch 1,
**dadurch gekennzeichnet,**
**dass** das Trägermaterial Aktivkohle, Siliciumcarbid, Aluminiumoxid, Siliciumoxid, Alumosilikat, Titandioxid, Zirkoniumdioxid, Magnesiumoxid und/oder Zinkoxid oder deren Gemische enthält.

3. Verfahren nach einem der Ansprüche 1 oder 2,
**dadurch gekennzeichnet,**
**dass** der Katalysator zusätzlich mindestens ein Metall der ersten Nebengruppe des Periodensystems der Elemente enthält.

4. Verfahren nach einem der Ansprüche 1 bis 3,
**dadurch gekennzeichnet,**
**dass** der Katalysator zusätzlich mindestens ein Metall der siebten Nebengruppe des Periodensystems der Elemente enthält.

5. Verfahren nach einem der Ansprüche 1 bis 4,
**dadurch gekennzeichnet,**
**dass** als aromatische Polycarbonsäureester Benzol-, Diphenyl-, Naphthalin-, Diphenyloxid- oder Anthracencarbonsäure-Ester eingesetzt werden.

## Claims

1. Process for catalytically hydrogenating alkyl esters, cycloalkyl esters and alcoxyalkyl esters of aromatic polycarboxylic acids to give the corresponding alicyclic polycarboxylic acid compounds, wherein the alcohol components of the esters are alkoxyalkyl groups, cycloalkyl groups, and/or alkyl groups having from 3 to 15 carbon atoms, branched or unbranched, and identical or different in each instance, using hydrogencontaining gases over a catalyst which comprises at least one metal of the eighth transition group of the periodic table on or in a support material,
**characterized in that** the support material has an average pore diameter of from 25 to 50 nm and a specific surface area greater than 30 m²/g, and over 90% of the total pore volume of the support materials is made up by meso- and micropores with a diameter of from 0.1 to 50 nm.

2. Process according to Claim 1,
**characterized in that**
the support material comprises activated carbon, silicon carbide, aluminum oxide, silicon oxide, aluminosilicate, titanium dioxide, zirconium dioxide, magnesium oxide, and/or zinc oxide, or a mixture of these.

3. Process according to Claim 1 or 2,
**characterized in that**
the catalyst also comprises at least one metal of the first transition group of the periodic table of the elements.

4. Process according to any of Claims 1 to 3,
**characterized in that**
the catalyst also comprises at least one metal of the seventh transition group of the periodic table of the elements.

5. Process according to any of Claims 1 to 4,
**characterized in that**
the aromatic polycarboxylic esters used comprise benzenecarboxylic esters, biphenylcarboxylic esters, naphthalenecarboxylic esters, diphenyl oxide carboxylic esters, or anthracenecarboxylic esters.

## Revendications

1. Procédé pour l'hydrogénation catalytique d'esters alkyliques, cycloalkyliques ainsi qu'alcoxyalkyliques d'acides polycarboxyliques aromatiques en composés de type acides polycarboxyliques alicycliques correspondants, les composants alcool des esters étant des groupes alcoxyalkyle, cycloalkyle et/ou alkyle comprenant 3 à 15 atomes de carbone, ramifiés ou non ramifiés, à chaque fois identiques ou différents, avec des gaz contenant de l'hydrogène sur un catalyseur, qui contient au moins un métal du huitième groupe secondaire du système périodique sur ou dans un matériau support, **caractérisé en ce que** le matériau support présente un diamètre moyen de pore de 25 à 50 nm et une surface spécifique supérieure à 30 m²/g et plus de 90% du volume total de pores du matériau support représentent des mésopores et des micropores d'un diamètre entre 0,1 et 50 nm.

2. Procédé selon la revendication 1, **caractérisé en ce que** le matériau support contient du charbon actif, du carbure de silicium, de l'oxyde d'aluminium, de l'oxyde de silicium, de l'aluminosilicate, du dioxyde de titane, du dioxyde de zirconium, de l'oxyde de magnésium et/ou de l'oxyde de zinc ou leurs mélanges.

3. Procédé selon l'une quelconque des revendications 1 ou 2, **caractérisé en ce que** le catalyseur contient en outre au moins un métal du premier groupe secondaire du système périodique des éléments.

4. Procédé selon l'une quelconque des revendications 1 à 3, **caractérisé en ce que** le catalyseur contient en outre au moins un métal du septième groupe secondaire du système périodique des éléments.

5. Procédé selon l'une quelconque des revendications 1 à 4, **caractérisé en ce qu'**on utilise comme esters d'acides polycarboxyliques aromatiques des esters d'acide benzènecarboxylique, d'acide diphényl-carboxylique, d'acide naphtalènecarboxylique, d'acide diphényloxydecarboxylique ou d'anthracènecarboxylique.
